# EUROPEAN PATENT APPLICATION

(11) **EP 4 356 891 A1**
(43) Date of publication of application: **24.04.2024**
(21) Application number: 22824558.5
(22) Date of filing: 10.03.2022
(51) Int. Cl.: A61F 13/496, A61F 13/53, A61F 13/535, A61F 13/536

(54) **DISPOSABLE DIAPER**

(30) Priority: 14.06.2021 JP 2021098758
(71) Applicant: Daio Paper Corporation, Ehime 799-0492 (JP)
(72) Inventor: TAKAGI, Yurika, Shikokuchuo-shi, Ehime 799-0113 (JP)
(74) Representative: Meissner Bolte Partnerschaft mbB
(86) International application number: PCT/JP2022/010689
(87) International publication number: WO 2022/264558

(57) **Abstract**

The problem is to attain, in a crotch part, both a fitting property of an absorbent element and shape maintainability of an absorber. The above problem is solved by a disposable diaper including an absorber element 50, which is provided in a range in a front-back direction LD including a crotch part M, and includes an absorbent 56 and a wrapping sheet 58 wrapping the absorber 56, wherein the absorber 56 is formed by accumulating a mixture of pulp fibers and super absorbent polymer particles, in the crotch part M, the absorber 56 has elongated low-basis weight sections 56L, which extend in the front-back direction LD on both sides thereof in a width direction WD, compressed portions 51, which are compressed in a thickness direction so as to be depressed from a surface into the absorber, are continuous in lattice shaped patterns provided across at least entire regions SR being lateral to the low-basis weight sections 56L in the absorbent element 50, and the wrapping sheet 58 is made of a nonwoven fabric having a bending resistance of 0.01 to 0.10 mN·cm in the front-back direction LD and 0.01 to 0.10 mN·cm in the width direction WD according to 41.5° Cantilever Method defined by JIS L 1913: 2010.

## Description

### Technical Field

The present invention relates to a disposable diaper where in a crotch part, a fitting property of an absorbent element and shape maintainability of an absorber are both attained.

### Background Art

In a disposable diaper, it is common to use an absorber formed by accumulating a mixture of pulp fibers and super absorbent polymer particles. Further, in general, such an absorber is incorporated in the disposable diaper in the form of an absorbent element in which the absorber is wound by a wrapping sheet made of a crepe tissue, etc. to improve shape maintainability of the absorber during manufacture and after manufacture (see Patent Literature 1, for example).

An absorbent element of a disposable diaper receives forces in various directions from both sides in a width direction due to movement of wearer's legs such as walking while being interposed between both the legs. Thus, in a crotch part, an excellent fitting property of the absorbent element is required, and further, in order to suppress shape deformation such as twisting and cracking of the absorber, shape maintainability is also required. In the disposable diaper, it is needless to say that, in the crotch part, an absorption amount of an absorbent element should be secured.

For example, in order to improve a fitting property of a crotch part, it is known that, in the crotch part, low-basis weight sections such as slits penetrating an absorber in a thickness direction are provided along a front-back direction on both sides thereof in the width direction. However, due to presence of such low-basis weight sections, forces are concentrated at both portions being lateral to the low-basis weight sections in the absorber such that the twisting and cracking thereof are tend to occur.

On the other hand, as a method for improving shape maintainability of an absorber, it is known that compressed portions formed by compressing an absorbent element in a thickness direction are provided in a lattice shaped pattern or the like (see Patent Literature 1, for example). However, when such compressed portions are provided, stiffness increase would be caused in the absorbent element. Accordingly, in order to improve the shape maintainability of the absorber, if it is done only that the compressed portions are provided in a pattern spread flatly such as the lattice shaped pattern on the absorbent element, the compressed portions are desirably provided so as not to reach both side portions of the absorbent element for preventing a decrease of the fitting property thereof.

### Citation List

### Patent Literature

Patent Literature 1: JP 2021-000236 A

### Summary of Invention

### Technical Problem

Therefore, a main object of the present invention is to attain, in a crotch part, both a fitting property of an absorbent element and shape maintainability of an absorber.

### Solution to Problem

A disposable diaper solving the above mentioned problem is as follows.

### <First aspect>

A disposable diaper including:
a crotch part;
a front side part which extends forward from the crotch part;
a back side part which extends backward from the crotch part; and
an absorbent element including an absorber and a wrapping sheet wrapping the absorber, the absorbent element being provided in a range in a front-back direction including the crotch part,
wherein the absorber is formed by accumulating a mixture of pulp fibers and super absorbent polymer particles,
in the crotch part, the absorber has elongated low-basis weight sections, which extend in the front-back direction on both sides thereof in a width direction,
compressed portions, which are compressed in a thickness direction so as to be depressed from at least one of a top surface and an underside surface of the absorbent element into the absorber, are continuous in lattice shaped patterns provided across at least entire regions being lateral to the low-basis weight sections in the absorbent element, and
the wrapping sheet is made of a nonwoven fabric having a bending resistance of 0.01 to 0.10 mN·cm in the front-back direction and 0.01 to 0.10 mN·cm in the width direction according to 41.5° Cantilever Method defined by JIS L 1913: 2010.

### (Effect)

The present disposable diaper is characterized in that
1) since the low-basis weight sections are provided, flexibility of the absorber is enhanced along these low-basis weight sections such that in the crotch part, a fitting property of the absorbent element can be improved,
2) due to the low-basis weight sections, twisting and cracking would tend to occur at portions being lateral to the low-basis weight sections in the absorber, but such twisting and cracking can be suppressed by the lattice shaped compressed portions provided across entire the regions being lateral to the low-basis weight sections, and
3) a fitting property of the crotch part would be decreased by the lattice shaped compressed portions, but such decrease can be suppressed by adopting the wrapping sheet being flexible, while an ability of the lattice shaped compressed portions to suppress the twisting and cracking is not impaired.

Therefore, according to the present disposable diaper, in the crotch part, the fitting property of the absorbent element and shape maintainability of the absorber are both attained more reliably, comparing with the conventional diapers.

### <Second aspect>

The disposable diaper according to the first aspect,
wherein the absorber has, in the crotch part, a narrowest portion having a width being 0.85 times to 1 time a maximum width of the front side part and the back side part.

### (Effect)

As a means for improving the fitting property of the crotch part, it is known that, in the crotch part thereof, an absorber is made to have a narrower portion by narrowing the absorber so as to have a narrower width of the narrower portion than those of both front and back sides thereof. However in this case, decrease of an absorption amount of the crotch part cannot be avoided. On the other hand, in a case where the absorber does not have such a narrower portion or the absorber has a narrower portion with a small constriction, although the absorption amount can be easily secured, the fitting property of the crotch part is decreased, and in addition, since the crotch part is likely to receive forces due to movement of wearer's legs, the shape maintainability of the absorber is likely to be decreased. To these problems, the above mentioned low-basis weight sections, lattice shaped compressed portions, and wrapping sheet made of the flexible nonwoven fabric are adopted in combination. By doing so, in the crotch part, the fitting property of the absorbent element and shape maintainability can be both attained while the absorption amount is secured. Note that in Patent Literature 1, an absorber has, in a crotch part, a narrowest portion having a width being 0.79 times a maximum width of a front side part and a back side part.

### <Third aspect>

The disposable diaper according to the first or second aspect,
wherein, in the absorber, the pulp fibers have a basis weight of 100 to 450 g/m², and
in the absorber, weight ratio of the pulp fibers : the super absorbent polymer particles is 50 : 50 to 20 : 80.

### (Effect)

As in the present aspect, it is preferable that the absorber has a high content of the super absorbent polymer particles from viewpoints of securement of the absorption amount and prevention of returning. In this case however, the twisting and cracking tend to occur at the portions being lateral to the low-basis weight sections in the absorber. To this problem, the above mentioned low-basis weight sections, lattice shaped compressed portions, and wrapping sheet made of the flexible nonwoven fabric are adopted in combination for such an absorber having the high content of the super absorbent polymer particles. By doing so, in the crotch part, the fitting property of the absorbent element and the shape maintainability can be both attained while an absorption amount and prevention of returning can be improved as a whole.

### <Fourth aspect>

The disposable diaper according to the third aspect,
wherein a width of each of the compressed portions is 1 to 3 mm, a thickness of each of the compressed portions is 15 to 35% of a maximum thickness of the absorbent element,
the compressed portions are formed in an oblique lattice including first portions each of which diagonally inclines at a clockwise angle of 40 to 50° with respect to the front-back direction in a plan view, and second portions each of which diagonally inclines at a counterclockwise angle of 40 to 50° with respect to the front-back direction in the plan view,
two or more unit frame rows in each of which unit frames including the compressed portions are repeated in the front-back direction are arranged in the width direction in at least a part of each of the regions being lateral to the low-basis weight sections.

### (Effect)

Dimensions and shapes of the compressed portions can be determined as appropriate, but it is preferable that the compressed portions have the structures as in the present aspect. In particular, in the present aspect, since the two or more unit frame rows each of which has the unit frames composed of the compressed portions are arranged in the width direction, the fitting property and shape maintainability of the absorbent element become excellent at the portions being lateral to the low-basis weight sections.

### <Fifth aspect>

The disposable diaper according to the fourth aspect,
wherein across at least entire the regions being lateral to the low-basis weight sections, an internal surface of the wrapping sheet is bonded to at least one of a top surface and an underside surface of the absorber on a side thereof, which has grooves of the compressed portions, by 5.0 to 20.0 g/m² of a hot melt adhesive.

### (Effect)

The shape maintainability of the absorbent element due to the compressed portions depends on maintainability of the compressed portions themselves. Further, this maintainability of the compressed portions themselves depends on the shape maintainability of the absorber itself and bonding strength between the wrapping sheet and the absorber in the compressed portions. Here, since a surface area of the absorber is increased due to the compressed portions formed thereon, a larger amount of the hot melt adhesive than a normal amount is required in order to secure sufficiently the bonding strength between the wrapping sheet and the absorber and the shape maintainability of the absorber itself. Therefore, as in the present aspect, not only that the internal surface of the wrapping sheet and an external surface of the absorber are bonded to each other by the hot melt adhesive, but also that, across at least the entire regions being lateral to the low-basis weight sections, the internal surface of the wrapping sheet is preferably bonded to at least one of the top surface and the underside surface of the absorber on the side thereof, which has the grooves of the compressed portions, by a sufficiently large amount of the hot melt adhesive.

### <sixth aspect>

The disposable diaper according to any one of the first to fifth aspects,
wherein the wrapping sheet has
an elongation rate of 20 to 100% in the front-back direction, measured according to a normal time method specified in JIS L 1913 : 2010, and
an elongation rate of 20 to 110% in the width direction, measured according to the normal time method specified in JIS L 1913 : 2010.

### (Effect)

When the wrapping sheet is elongated with ease which is similar level to that of the present aspect, flexibility of the absorbent element is improved while an ability of the lattice shaped compressed portions to suppress the twisting and cracking is not impaired, so that in the crotch part, the fitting property of the absorbent element is improved.

### Advantageous Effects of Invention

According to the present invention, in a crotch part, a fitting property of an absorbent element and shape maintainability of an absorber can be both attained.

### Brief Description of Drawings

Fig. 1 is a plan view illustrating an internal surface of an underpants-type disposable diaper in a spread state.
Fig. 2 is a plan view illustrating an external surface of the underpants-type disposable diaper in the spread state.
Fig. 3 is a cross-sectional view taken along 2-2 line of Fig. 1.
Fig. 4 is a cross-sectional view taken along 3-3 line of Fig. 1.
Fig. 5(a) is a cross-sectional view taken along 4-4 line of Fig. 1, and Fig. 5(b) is a cross-sectional view taken along 5-5 line of Fig. 1.
Fig. 6 is a perspective view of the underpants-type disposable diaper.
Fig. 7 is a plan view illustrating an external surface of an inner member together with an outline of an outer member in the spread state.
Fig. 8 is a plan view illustrating a top surface of an absorber together with an outline of a wrapping sheet.
Fig. 9 is a cross-sectional view of an absorbent element.
Fig. 10 is a plan view of the absorbent element.
Fig. 11 is an enlarged view of a main part of a top surface of the absorbent element.
Fig. 12 is a plan view illustrating other examples of the absorber.
Fig. 13 is a cross-sectional view illustrating the absorbent element in a state where it is before the absorber is wrapped with the wrapping sheet.
Fig. 14 is a cross-sectional view illustrating the absorbent element in a state where it is after the absorber is wrapped with the wrapping sheet and before compressed portions are formed.

### Description of Embodiments

Hereinafter, a detailed description will be given of an underpants-type disposable diaper as an example of a disposable diaper, referring to accompanying drawings. Fixing or bonding of respective constituent members adjacent to each other in a thickness direction other than fixed portions or bonded portions described below may be carried out in the same manner as those for known diapers as necessary. A dotted pattern portion in each of the cross-sectional views designates an adhesive such as a hot melt adhesive as a fixing or bonding means. The hot melt adhesive can be applied by a known method such as slot application, bead application into a continuous line or dot line, spray application into a spiral shape, a Z shape, or a wave shape, or by pattern coating (transfer of a hot melt adhesive by a letterpress method). Alternatively, the fixed portion of an elastic member is formed, instead of or in addition to the above applications of the hot melt adhesive, by application of the hot melt adhesive to an outer peripheral surface of the elastic member, and the elastic member can be fixed to a member located adjacent thereto. Examples of the hot melt adhesive include an EVA-based agent, a pressure sensitive adhesive rubber-based agent (elastomer-based agent), a polyolefin-based agent, and a polyester/polyamide-based agent, and these can be used without particular limitation. As a fixing or bonding means for bonding the respective constituent members to each other, a means by material welding such as heat sealing or ultrasonic sealing also can be used. For a part requiring liquid perviousness in the thickness direction, the constituent members adjacent to each other in the thickness direction are fixed or bonded to each other in an intermittent pattern. For example, in a case where such fixing or bonding in the intermittent pattern is carried out by the hot melt adhesive, the application in the intermittent pattern of a spiral shape, a Z shape, or a wave shape can be carried out preferably. Then, in a case where an application range of the hot melt adhesive has a width being the same as or larger than that of an application range of the hot melt adhesive come out from one nozzle, this application can be carried out in an intermittent pattern of a spiral shape, a Z shape, or a wave shape formed continuously or at intervals in a width direction. As a bonding means for bonding the respective constituent members to each other, a means by material welding such as heat sealing or ultrasonic sealing also can be used.

Further, as a nonwoven fabric in the following description, a known nonwoven fabric can be appropriately used according to a site or a purpose. Examples of a constituent fiber of the nonwoven fabric include, but are not limited to, a synthetic fiber such as polyolefin-based fiber including polyethylene and polypropylene, a polyester-based fiber, or a polyamide-based fiber (including a composite fiber such as core-sheath in addition to a single component fiber), a regenerated fiber such as rayon or cupra, or a natural fiber such as cotton, and also mixture thereof. In order to enhance flexibility of the nonwoven fabric, it is preferable to use a crimped fiber as the constituent fiber. In addition, the constituent fiber of the nonwoven fabric may be a hydrophilic fiber (including a fiber that has become hydrophilic by a hydrophilizing agent), a hydrophobic fiber, or a water-repelling fiber (including a fiber that has become water-repellent by a water repellent agent). Further, the nonwoven fabric is generally classified into a short fiber nonwoven fabric, a long fiber nonwoven fabric, a spunbond nonwoven fabric, a meltblown nonwoven fabric, a spunlace nonwoven fabric, a thermal bond (air-through) nonwoven fabric, a needle punch nonwoven fabric, a point bond nonwoven fabric, a complex nonwoven fabric (including an SMS nonwoven fabric, an SMMS nonwoven fabric, or the like in each of which a meltblown layer is interposed between spunbond layers), and the like depending on a fiber length, a sheet forming method, a fiber bonding method, and a stacked structure, and any of these nonwoven fabrics can be used.

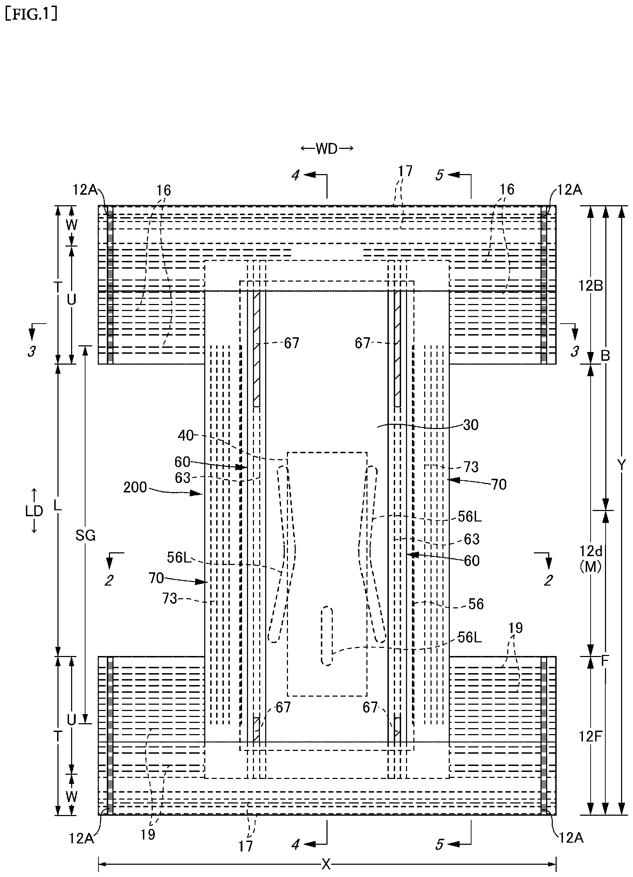

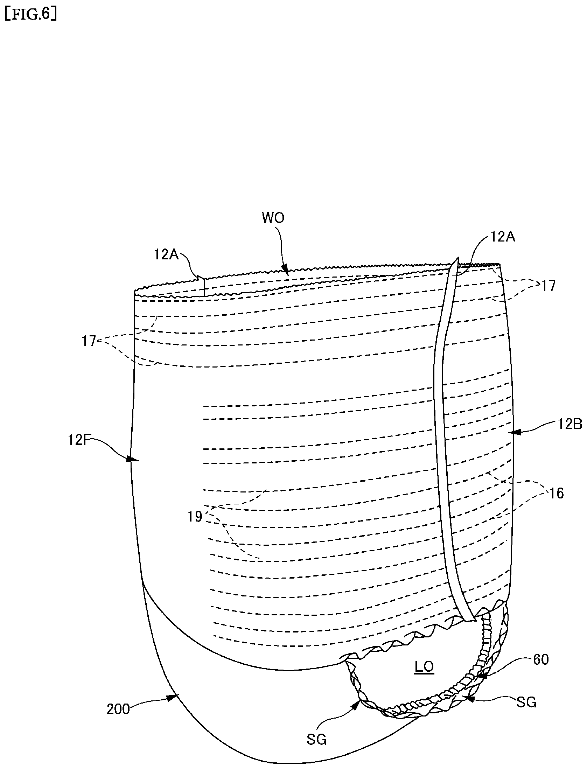

Fig. 1 to Fig. 6 illustrate an example of underpants-type disposable diaper. The present underpants-type disposable diaper includes a rectangular shaped front outer member 12F forming a lower torso portion on a front side, a rectangular shaped back outer member 12B forming the lower torso portion on a back side, and an inner member 200 disposed inside an outer member 12F, 12B so as to extend from the front outer member 12F to the back outer member 12B through a crotch part M. Both sides of the front outer member 12F and both sides of the back outer member 12B are bonded to each other to form side seal portions 12A. Thus, an opening formed by front and back end portions of the front and back outer member 12F, 12B acts as a waist opening WO, through which a wearer's torso passes, and both portions surrounded by lower edges of the outer member 12F, 12B and side edges of the inner member 200 on both sides thereof in the width direction act as leg openings LO, through which wearer's legs pass. The inner member 200 refers to a portion for absorbing and holding excrement such as urine, and the outer member 12F, 12B refer to a portion for supporting the inner member 200 with respect to a wearer's body. In addition, a reference sign Y designates a maximum length of the diaper in a spread state (a length from an edge of the waist opening WO in a front body F to an edge of the waist opening WO in a back body B in a front-back direction), and a reference sign X designates a maximum width of the diaper in the spread state.

The present underpants-type disposable diaper includes a lower torso region T defined as a range in the front-back direction (a range in the front-back direction from the waist opening WO to upper ends of the leg openings LO) having the side seal portions 12A, and an intermediate region L defined as a range in the front-back direction having a portion forming the leg openings LO (between a region in the front-back direction having the side seal portions 12A of the front body F and a region in the front-back direction having the side seal portions 12A of the back body B). A portion located on the lower torso region T in the front outer member 12F and the back outer member 12B, that is, the lower torso portion in the front side and the back side can be divided into a "waist end portion" W conceptually forming an edge of the waist opening and an "under-waist end portion" U which is a portion lower than the waist end portion W. Usually, in a case where the lower torso region T has a boundary in which a stretching stress in the width direction WD changes (for example, the fineness of an elastic member or the stretch rate thereof changes), a portion closer to the waist opening WO than the boundary closest to the waist opening WO refers to the waist end portion W. In a case where there is no such a boundary, a waist opening side extending portion 12E, which is closer to the waist opening WO than the absorber 56 or the inner member 200, refers to the waist end portion W. Lengths in the front-back direction of these portions vary depending on the size of a product and can be appropriately determined, for example, the length in the front-back direction of the waist end portion W can be 15 to 40 mm, and the length in the front-back direction of the under-waist end portion U can be 65 to 120 mm. Meanwhile, the intermediate region L is narrowed on both side edges in substantially U shapes or curved shapes so as to follow peripheries of the wearer's legs, and both the side edges correspond to sites through which the legs are inserted. Therefore, the underpants-type disposable diaper in the spread state has a substantially hourglass shape as a whole.

### (Inner member)

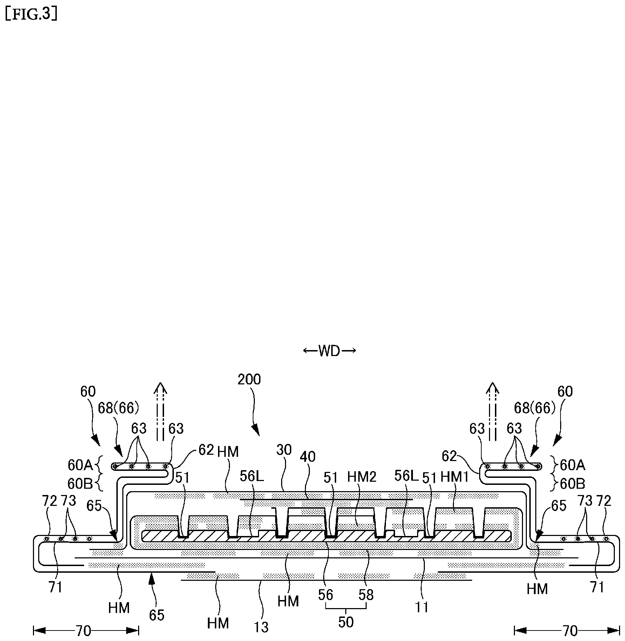

The inner member 200 can adopt an arbitrary shape, but has a rectangular shape in the illustrated example. As illustrated in Figs. 3 to 5, the inner member 200 includes a top sheet 30 on a body side; a liquid impervious sheet 11; and an absorbent element 50 disposed therebetween, and is a main unit portion for performing an absorption function. A reference sign 40 designates an intermediate sheet (second sheet) disposed between the top sheet 30 and the absorbent element 50 in order to rapidly transfer a liquid that has passed through the top sheet 30 to the absorbent element 50. Reference signs 60 on both sides designate rising gathers, which rise from both side portions of the inner member 200 so as to come into contact with the peripheries of the wearer's legs in order to prevent leakage of excrement into both sides of the inner member 200.

### (Top sheet)

The top sheet 30 transmits a liquid, and examples thereof include a perforated or imperforated nonwoven fabric, or a porous plastic sheet. In addition, the top sheet 30 may be composed of a single sheet or a stacked sheet obtained by adhering two or more sheets to each other. Similarly, the top sheet 30 may be composed of a single sheet or two or more sheets in a plane direction.

Both side portions of the top sheet 30 may be folded back to an underside of the absorbent element 50 at side edges thereof, or may extend laterally beyond the side edges of the absorbent element 50 without being folded back.

It is desirable that the top sheet 30 is fixed to members adjacent to the top sheet 30 on underside thereof by the hot melt adhesive or a bonding means by material welding such as heat sealing or ultrasonic sealing, for the purpose of preventing positional deviation of the top sheet 30 with respect to the members on the underside of the top sheet 30. In the illustrated example, the top sheet 30 is fixed to a top surface of the intermediate sheet 40 and a top surface of a wrapping sheet 58 at portions thereof located on a top side of the absorber 56, by the hot melt adhesive applied to an underside surface of the top sheet 30.

### (Intermediate sheet)

In order to quickly transfer a liquid that has passed through the top sheet 30 to the absorber, it is possible to dispose the intermediate sheet (also referred to as "second sheet") 40 having a higher liquid transmission rate than the top sheet 30. The intermediate sheet 40 is used in order to rapidly transfer a liquid to the absorber to enhance absorption performance of the absorber, and to prevent a "returning" phenomenon of the absorbed liquid from the absorber. The intermediate sheet 40 can be omitted.

Examples of the intermediate sheet 40 may be formed of a similar material to that of the top sheet 30, a spun lace nonwoven fabric, a spunbond nonwoven fabric, a SMS nonwoven fabric, a pulp nonwoven fabric, a mixed sheet of pulp and rayon, a point bond nonwoven fabric or crepe tissue. In particular, an air-through nonwoven fabric is bulky, and thus is preferable. It is preferable to use a composite fiber having a core-sheath structure for the air-through nonwoven fabric. In this case, a resin used for a core may be polypropylene (PP). However, polyester (PET) having high rigidity is preferable. A basis weight is preferably 17 to 80 g/m², more preferably 25 to 60 g/m². A fineness of a raw material fiber of the nonwoven fabric is preferably 2.0 to 10 dtex. In order to increase the bulkiness of the nonwoven fabric, it is preferable to use an eccentric fiber having no core in a center, a hollow fiber, and an eccentric and hollow fiber as a mixed fiber of a whole or a part of the raw material fiber.

The intermediate sheet 40 in the illustrated example is disposed at the center so as to be shorter than a width of the absorber 56, but may be disposed over a maximum width of the absorber 56. A length in the front-back direction of the intermediate sheet 40 may be the same as the maximum length of the diaper, may be the same as a maximum length of the absorbent element 50, or may be within a range having a short length centered on a region in which a liquid is received.

It is desirable that the intermediate sheet 40 is fixed to members adjacent to the intermediate sheet 40 on underside thereof by the hot melt adhesive or a bonding means by material welding such as heat sealing or ultrasonic sealing for the purpose of preventing positional deviation of the intermediate sheet 40 with respect to the members on the underside of the intermediate sheet 40. In the illustrated example, the intermediate sheet 40 is fixed to the top surface of the wrapping sheet 58 at portions thereof located on the top side of the absorber 56, by the hot melt adhesive applied to an underside surface of the intermediate sheet 40.

### (Liquid impervious sheet)

A material of the liquid impervious sheet 11 is not particularly limited, but examples thereof include a plastic film formed of a polyolefin-based resin such as polyethylene or polypropylene, a laminated nonwoven fabric having a plastic film disposed on a surface of a nonwoven fabric, and a stacked sheet obtained by superposing and bonding a nonwoven fabric or the like to a plastic film. For the liquid impervious sheet 11, it is preferable to use a liquid impervious and moisture pervious material favorably used from a viewpoint of preventing stuffiness. As a moisture pervious plastic film, a microporous plastic film is widely used which is obtained by kneading an inorganic filler in a polyolefin resin such as polyethylene or polypropylene, molding the kneaded mixture into a sheet, and then stretching the sheet in one or two axial directions. In addition, a nonwoven fabric using a micro denier fiber, a nonwoven fabric that has reinforced leakproofness by reducing a space between fibers by heating or applying pressure, and a sheet that has become liquid impervious without using a plastic film by a method for applying a super absorbent polymer, a hydrophobic resin, or a water repellent agent can be used as the liquid impervious sheet 11. However, it is desirable to use a moisture pervious plastic film in order to obtain sufficient bonding strength at the time of bonding to a cover nonwoven fabric 13 described later by the hot melt adhesive.

The liquid impervious sheet 11 may have a width housed in an underside of the absorbent element 50 as illustrated in the drawing, or may go around both sides of the absorbent element 50 and extend to the both side portions on a surface of the absorbent element 50 on a top sheet 30 side in order to enhance leakproofness. Thus, an extending portion formed in this way has appropriately a width of about 5 to 20 mm on each of the left and the right.

### (Absorbent element)

The absorbent element 50 includes the absorber 56 and the wrapping sheet 58 wrapping a whole of the absorber 56.

### (Absorber)

The absorber 56 is obtained by accumulating a mixture of pulp fibers and super absorbent polymer particles. A fiber basis weight of the absorber 56 may be set to about 100 to 450 g/m².

The super absorbent polymer particles include "powder" in addition to "particles". As super absorbent polymer particles, those used for this type of disposable diaper can be used as they are. For example, when sieving using a standard sieve of 500 um (JIS Z8801-1: 2006) (shake for five minutes) is performed, particles in which a ratio of particles remaining on the sieve is 30% by weight or less are desirable. When sieving using a standard sieve of 180 um (JIS Z8801-1: 2006) (shake for five minutes) is performed, particles in which a ratio of particles remaining on the sieve is 60% by weight or more are desirable.

A material of the super absorbent polymer particles can be used without particular limitation, but those having a water absorption capacity of 40 g/g or more are preferable. Examples of the super absorbent polymer particles include a starch-based material, a cellulose-based material, and a synthetic polymer-based material. A starch-acrylic acid (salt) graft copolymer, a saponified product of a starch-acrylonitrile copolymer, a cross-linked product of sodium carboxymethyl cellulose, an acrylic acid (salt) polymer, or the like can be used. As the shapes of the super absorbent polymer particles, a usually used particulate material shape is suitable, but other shapes can also be used.

As the super absorbent polymer particles, those having a water absorption rate of 70 seconds or less, particularly 40 seconds or less are suitably used. When the water absorption rate is too slow, so-called returning that a liquid supplied into the absorber 56 returns out of the absorber 56 tends to occur.

As the super absorbent polymer particles, those having a gel strength of 1000 Pa or more are suitably used. This makes it possible to effectively suppress sticky feeling after liquid absorption even in a case of using the bulky absorber 56.

The basis weight of the super absorbent polymer particles can be appropriately determined depending on an absorption amount required for a use application of the absorber 56. Therefore, the basis weight can be 50 to 350 g/m² although this cannot be applied generally. The basis weight of the super absorbent polymer particles of less than 50 g/m² makes it difficult to secure the absorption amount. The basis weight of more than 350 g/m² saturates an effect.

A ratio of fibers to super absorbent polymer particles in the absorber 56 is not particularly limited. However, when a weight ratio of fibers : super absorbent polymer particles corresponds to 50 : 50 to 20 : 80, and when comparison is performed at the same area and the same absorption amount, the thinner absorber 56 may be obtained. In this case, a thickness 56t of the absorber 56 is not particularly limited. However, the thickness 56t may be set to 3 to 15 mm.

It is enough that the absorber 56 extends to both front and back sides of the crotch part M so as to include it. In the underpants-type disposable diaper as in the present example, the absorber 56 preferably extends to a peripheral edge of the inner member 200 or the vicinity thereof in the front-back direction LD and the width direction WD. Note that a reference sign 56X designates the maximum width of the absorber 56.

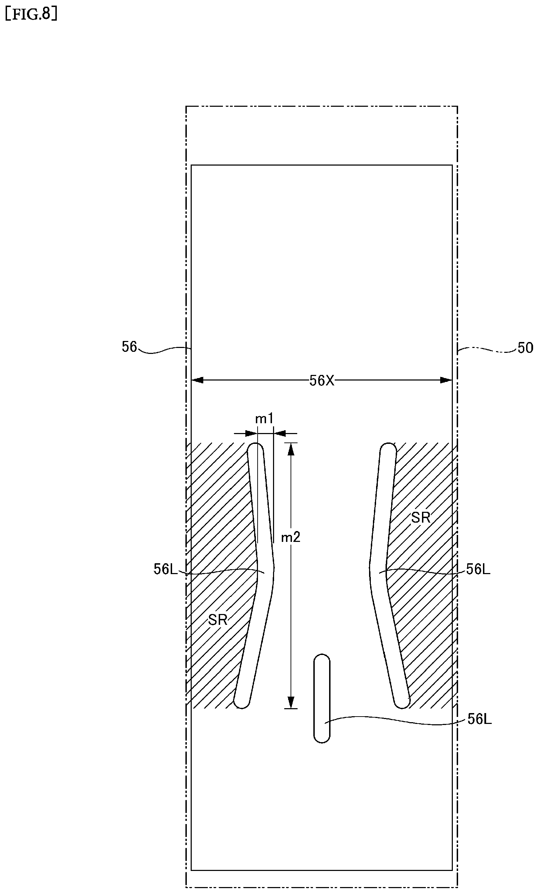

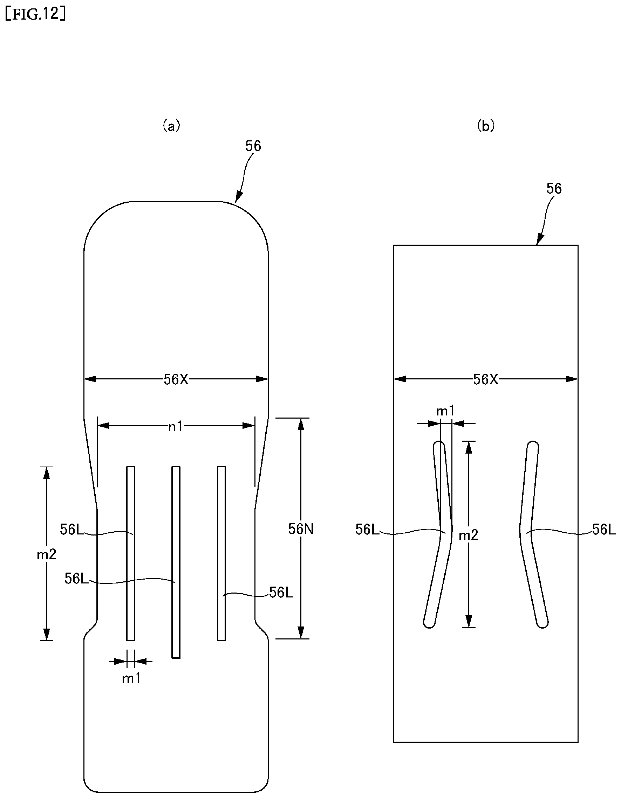

In order to make it easy to secure an absorption amount of the crotch part M, the absorber 56 may be preferably formed into a substantially rectangular shape as in the example illustrated in Fig. 8. In addition, as in the example illustrated in Fig. 12(a), in order to improve a fitting property of the crotch part M, it is possible that in the crotch part M, the absorber 56 is made to have a narrower portion by narrowing the absorber 56 so as to have a narrower width of the narrower portion than those of both front and back sides thereof. In this case, in order to make it easy to secure the absorption amount of the crotch part M, it is preferable that the absorber 56 has, in the crotch part M, a narrowest portion having a narrowest width n1 being equal to or more than 0.85 times the maximum width 56X of the absorber 56.

Incidentally, in a case where the absorber 56 has the narrower portion 56N, the crotch part M refers to a range in the front-back direction LD having the narrower portion 56N; in a case where the absorber 56 does not have the narrower portion 56N, and instead, as in the illustrated example, an outer shape of the diaper in the spread state has a narrower portion, the crotch part M refers to a range in the front-back direction LD having this narrower portion of the outer shape (in the illustrated example, the crotch part M is disposed between the front outer member 12F and the back outer member 12B); and in a case where the absorber 56 has neither of such narrowing portions, the crotch part M refers to a part disposed at a center in the front-back direction LD and a dimension in the front-back direction LD thereof is 20 to 30% of a maximum length of the product. A part extending forward from the crotch part M and a part extending backward therefrom refer to a front side part and a back side part respectively.

### (low-basis weight section)

In the crotch part M, the absorber 56 has elongated low-basis weight sections 56L, which extend in the front-back direction LD on both sides thereof in the width direction WD. The low-basis weight section 56L refers to a section having a low basis weight and excludes a portion, which is just compressed in the thickness direction and whose basis weight is not changed as can be seen in a compressed portion 51 discussed later. As the low-basis weight section 56L, a slit, which penetrates the absorber in the thickness direction, can be adopted. However, as in the illustrated example, a recess in which a small amount of the mixture of the pulp fibers and the super absorbent polymer particles are accumulated is preferable as the low-basis weight section 56L because of easiness in securing the absorption amount. This recess may be formed on a top surface of the absorber or on an underside surface of the absorber. Since the low-basis weight sections 56L are provided in the absorber 56, flexibility of the absorber 56 is enhanced along these low-basis weight sections 56L such that in the crotch part M, a fitting property of the absorbent element 50 can be improved. It is enough only that the total basis weight of the pulp fibers and the super absorbent polymer particles in the low-basis weight sections 56L is smaller than the total basis weight thereof in a portion of the absorber other than the low-basis weight sections 56L. For example, it is possible that the total basis weight thereof in the low-basis weight sections 56L is set to be 0.1 to 0.5 times the total basis weight thereof in the portion of the absorber other than the low-basis weight sections 56L.

As long as the low-basis weight section 56L extends in the front-back direction LD so as to be elongated, the low-basis weight section 56L may extend linearly along the front-back direction LD, or as in the illustrated example, the low-basis weight section 56L may be curved so that it is positioned laterally with increasing proximity to each end thereof along the front-back direction LD. Further, a front end portion and a back end portion of the low-basis weight section 56L can have appropriate shapes. For example, the low-basis weight section 56L has a linear shape as a whole as illustrated in Fig. 12(a). Alternatively, both the front end portion and the back end portion of the low-basis weight section 56L have convex shapes formed with curved lines (arcs of semi-circles, or the like) as in an example illustrated in Fig. 8, or the low-basis weight section 56L has, in each of the front end portion and the back end portion, two round corners and a linear shaped part therebetween, although not illustrated. A width m1 of the low-basis weight section 56L can be determined as appropriate, and for example, the width m1 can be 0.04 to 0.1 times the narrowest width n1 of the narrowest portion of the absorber 56 in the crotch part M (if the absorber 56 has a rectangular shape, the width n1 refers to the maximum width 56X). The width m1 of the low-basis weight section 56L can be constant or can be changed in a length direction thereof. A dimension in the front-back direction LD and an arrangement of the low-basis weight section 56L can be determined as appropriate. For example, a dimension m2 in the front-back direction LD of the low-basis weight section 56L can be 50 to 120%, preferably 50 to 80% of a dimension in the front-back direction LD of the crotch part M. Further, the low-basis weight section 56L may be housed in a range of the crotch part M, or may extend beyond the range of the crotch part M to the front side, the back side, or both the front and back sides thereof.

As long as, in the crotch part M, one low-basis weight section 56L is provided on each of both (right and left) sides of the absorber 56 in the width direction WD, three or more low-basis weight sections 56L in total can be provided, such as an embodiment where one low-basis weight section 56L is added so as to locate at a center in the width direction in addition to both the low-basis weight sections 56L provided on both the sides, as illustrated in Fig 8 and Fig. 12(a), alternatively, only two sections 56L in total may be provided as illustrated in Fig. 12(b).

### (Compressed portion)

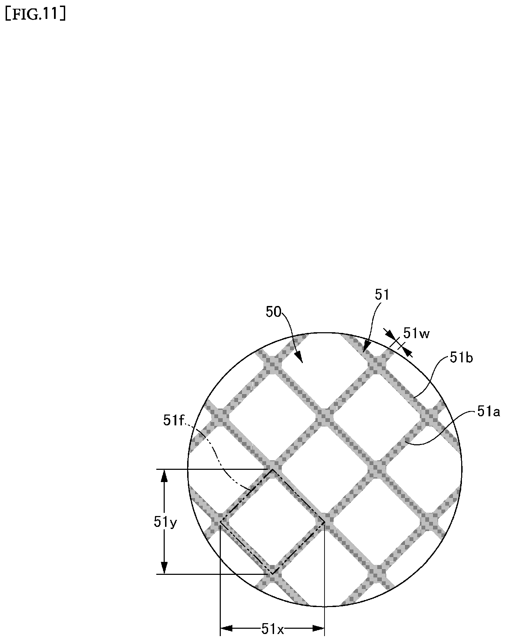

In the absorbent element 50, as illustrated in Fig. 3, Fig. 4 and Fig. 9, compressed portions 51, which are compressed in the thickness direction so as to be depressed from at least one of a top surface and an underside surface of the absorbent element 50 into the absorber 56, are continuous in lattice shaped patterns provided across at least entire regions SR being lateral to the low-basis weight sections 56L (see Fig. 8). When such lattice shaped compressed portions 51 are provided, the pulp fibers and the super absorbent polymer particles are constrained within respective unit frames 51f (see Fig. 11) including the compressed portions 51 such that twisting and cracking of the absorber 56 can be suppressed. In particular, when the above mentioned low-basis weight sections 56L are provided, the twisting and cracking would tend to occur at the portions being lateral to the low-basis weight sections 56L in the absorber 56. However, the twisting and the cracking can be suppressed efficiently by the lattice shaped compressed portions 51, which are provided across the entire regions being lateral to the low-basis weight sections.

As long as the lattice shaped compressed portions 51 are continuous in the lattice shaped patterns provided across entire the regions SR being lateral to the low-basis weight sections 56L, the compressed portions 51 may be provided in the absorbent element 50 only at ranges including entire the above regions SR, or may be provided all over the absorbent element 50 in a continuous lattice shaped pattern, as in the illustrated example. The lattice shaped compressed portions 51 can be formed by pressing the absorbent element 50 in the lattice shaped pattern from a top side, an underside, or both of the top side and the underside thereof by means of non-thermal embossing or hot embossing.

A width 51w of the compressed portion 51 (a width of a bottom portion of a groove formed on the absorbent element 50) can be determined as appropriate, but in a normal case, the width 51w is preferably about 1 to 3 mm.

A thickness 51t of the compressed portion 51 can be determined as appropriate, but in a normal case, the thickness 51t is preferably 15 to 35% of a maximum value of the thickness 50t of the absorbent element 50.

A shape of a unit frame 51f is not particularly limited and may be a substantial square shape as in the illustrated example, in addition to this shape, may be another polygonal shape such as a substantial rhombus shape (excluding a square shape), a substantial rectangular shape, , and a substantial triangle shape. Further, as long as the lattice shaped compressed portions 51 are provided in the lattice shaped pattern, shapes of the unit frames 51f partly may be different each other.

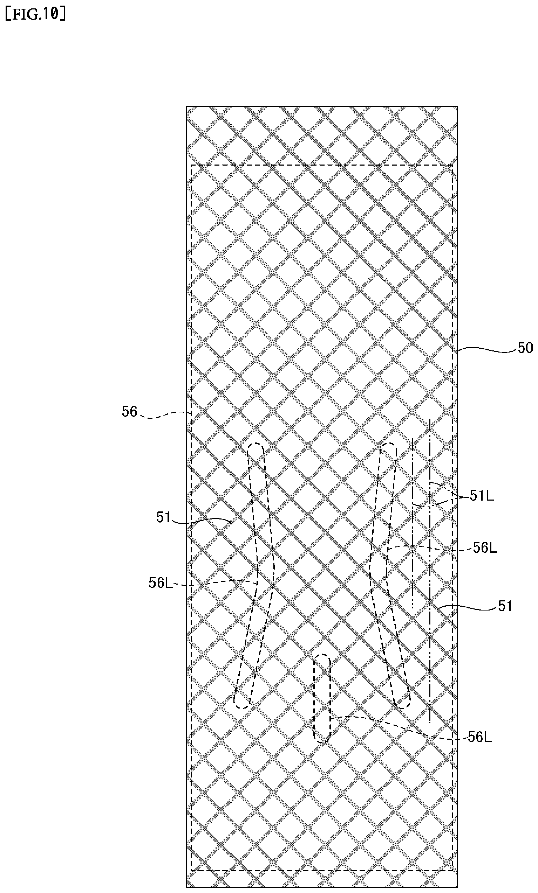

In one preferable pattern of the compressed portions 51, as the example illustrated in Fig. 10 and Fig. 11, the compressed portions 51 include first portions 51a each of which extends diagonally in a direction inclining at a clockwise angle of 40 to 50° with respect to the front-back direction LD in a plan view, and second portions 51b each of which extends diagonally in a direction inclining at a counterclockwise angle of 40 to 50° with respect to the front-back direction LD in the plan view such that the compressed portions 51 have an oblique lattice shaped pattern. In this case, shape of the unit fame 51f becomes a substantial rhombus shape.

Dimensions of the unit frames 51f can be determined as appropriate. However, it is preferable that the dimensions are nearly set, on the basis of a fact that complete unit frames 51f are repeated in the front-back direction LD in a unit frame row 51L, so as to allow two or more unit frame rows 51L to be arranged in the width direction WD in at least a part of each of the regions being lateral to the low-basis weight sections 56L. In this way, the fitting property and shape maintainability of the absorbent element 50 become excellent at the portions being lateral to the low-basis weight sections 56L. More specifically, a dimension 51x in the width direction WD of the unit frame 51f can be about 15 to 20 mm. In addition, a dimension 51y in the front-back direction LD of the unit frame 51f can be about 15 to 20 mm.

### (Wrapping sheet)

As the wrapping sheet 58, a nonwoven fabric is used which has a bending resistance of 0.01 to 0.10 mN·cm in the front-back direction LD and 0.01 to 0.10 mN·cm in the width direction WD according to 41.5° Cantilever Method defined by JIS L 1913: 2010. The fitting property of the crotch part M would be decreased by the lattice shaped compressed portions 51, but such decrease can be suppressed by adopting the wrapping sheet being flexible, while an ability of the lattice shaped compressed portions 51 to suppress the twisting and cracking is not impaired. Therefore, in the crotch part M, the fitting property of the absorbent element 50 and shape maintainability of the absorber 50 are both attained. It is more preferable that the nonwoven used for the wrapping sheet 58 has a bending resistance of 0.03 to 0.05 mN·cm in the front-back direction LD and 0.01 to 0.04 mN·cm in the width direction WD.

The nonwoven fabric used for the wrapping sheet 58 is not particularly limited, but an SMS nonwoven fabric, an SSMMS nonwoven fabric, or the like can be used favorably in each of which at least one meltblown layer is interposed between a pair of top side and underside spunbond layers. A material of the fibers of the nonwoven fabric is not particularly limited. For example, as the material, polypropylene fibers and polyethylene/polypropylene bicomponent fibers or the like can be used. A basis weight of the wrapping sheet 58 can be determined as appropriate, but the basis weight of 5 to 40 g/m², particularly of 10 to 30 g/m² is desirable.

In a case where the wrapping sheet 58 is elongated with ease, it becomes easy not only to form the compressed portions 51, but also to maintain the compressed portions 51 even when the absorbent element 50 is deformed by an external force applied in a wearing state. Therefore, flexibility of the absorbent element 50 is improved while an ability of the lattice shaped compressed portions 51 to suppress the twisting and cracking is not impaired, such that in the crotch part M, the fitting property of the absorbent element 50 can be improved. It is, therefore, preferable that the wrapping sheet 58 has an elongation rate measured according to a normal time method specified in JIS L 1913 : 2010 of 20 to 100%, particularly 40 to 60%, in the front-back direction LD, and an elongation rate measured according to the normal time method specified in JIS L 1913 : 2010 of 20 to 110%, particularly 50 to 70%, in the width direction WD.

A wrapping mode with the wrapping sheet 58 may be determined appropriately. However, in view of ease of manufacturing and in view of limiting escape of the super absorbent polymer particles from front and back end edges of the wrapping sheet 58, the following mode is preferable. Precisely, the wrapping sheet 58 wraps around the absorber 56 in a tubular shape so as to surround the top surface, the underside surface, and both side surfaces thereof, while a front end edge portion and a back end edge portion of the wrapping sheet 14 are made to protrude forward and backward from the absorber 56 respectively. It is preferable that the overwrapped portions of the wrapping sheet 58 at the seam and the overwrapped portions of the wrapping sheet 58 protruded forward and backward from the absorber 56, respectively, are bonded to each other by the hot melt adhesive or the bonding means by material welding.

The shape maintainability of the absorbent element 50 due to the compressed portions 51 depends on maintainability of the compressed portions 51 themselves. Further, this maintainability of the compressed portions 51 themselves depends on the shape maintainability of the absorber 56 itself and bonding strength between the wrapping sheet 58 and the absorber 56 in the compressed portions 51. Then, in a case where the wrapping sheet 58 and the absorber 56 are bonded to each other by hot melt adhesives HM1 and HM2, since a surface area of the absorber is increased due to the compressed portions 51 formed thereon, a larger amount of the hot melt adhesives HM1 and HM2 than a normal amount is required in order to secure sufficiently the bonding strength between the wrapping sheet 58 and the absorber 56 and the shape maintainability of the absorber 56 itself. Therefore, it is preferable that across at least entire the regions SR being lateral to the low-basis weight sections 56L, the internal surface of the wrapping sheet 58 and at least one of the top surface and the underside surface of the absorber 56 on a side thereof, which has grooves of the compressed portions 51, are bonded to each other by 5.0 to 20.0 g/m², particularly 7.5 to 15.0 g/m² of the hot melt adhesives HM1, HM2.

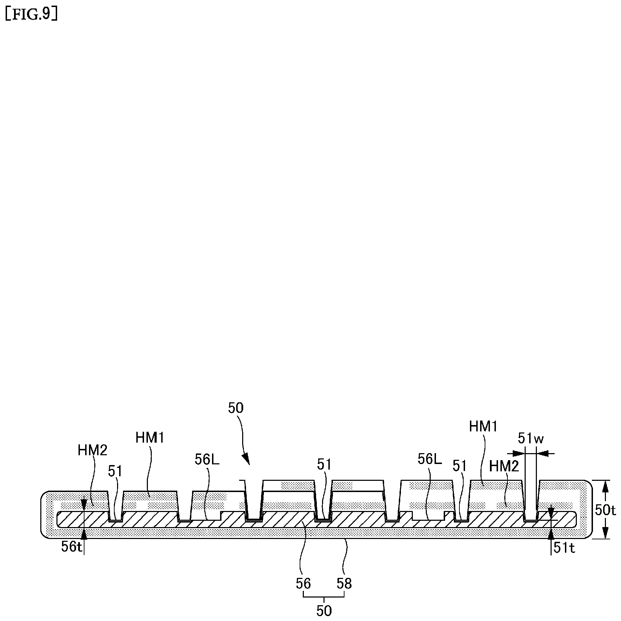

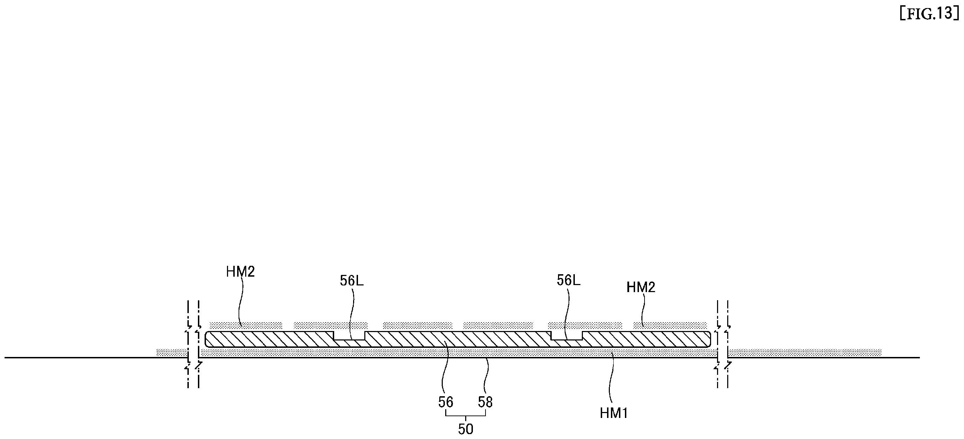

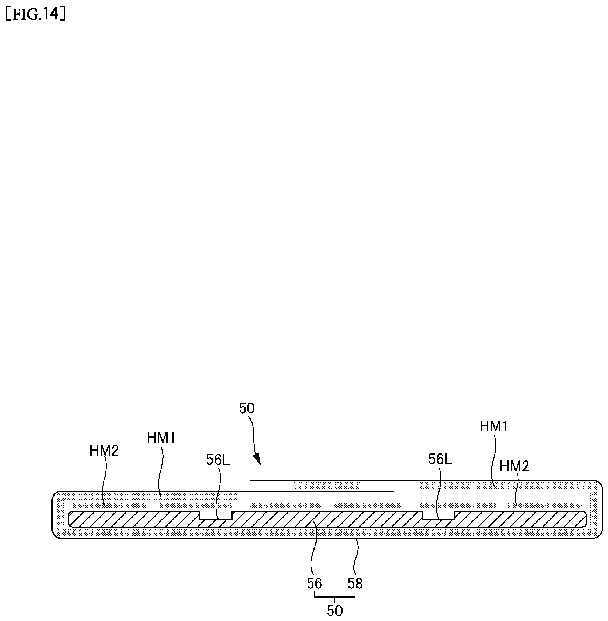

For example, as illustrated in Fig. 9, in a case where the grooves are formed by the compressed portions 51 on the top surface of the absorber 56, one layer of the hot melt adhesive HM1 and another layer of the hot melt adhesive HM2 are provided on the top surface of the absorber 56 in both the sides thereof in the width direction WD such that these layers have widths including at least entire the regions SR being lateral to the low-basis weight sections 56L. In this way, the above mentioned amount of the hot melt adhesives HM can be attained. Such structure is formed as follows. First, as illustrated in Fig. 13, the first hot melt adhesive HM1 is applied over a substantially entire surface facing the absorber 56 of the internal surface of the wrapping sheet 58 in the spread state. After that, the absorber 56, which has already the low-basis weight sections 56L formed thereon, is disposed on the first hot melt adhesive HM1 applied on the wrapping sheet 58 at an intermediate portion thereof in the width direction WD, such that the underside surface of the absorber 56 and the wrapping sheet 58 are bonded to each other by the first hot melt adhesive HM1. Then, the second hot melt adhesive HM2 is applied over the substantially entire top surface of the absorber 56. After that, as illustrated in Fig. 14, protruded portions of the wrapping sheet 58 from both side edges of the absorber 56 are folded back to the top surface of the absorber 56, as folded portions of the wrapping sheet 58. In this way, these folded portions of the wrapping sheet 58 and the top surface of the absorber 56 are bonded to each other by the first hot melt adhesive HM1 and the second hot melt adhesive HM2. In addition, the overlapped portions of the wrapping sheet 58 are bonded to each other by the first hot melt adhesive HM1. Finally, as illustrated in Fig. 9, the lattice shaped compressed portions 51 are formed by embossing.

### (Rising gather)

The rising gather 60 has a rising portion 68 rising from a side of the inner member 200, and the rising portion 68 comes into contact with a range of the wearer's body from an inguinal portion to a gluteal region through a periphery of the leg in order to prevent side leakage. In the rising gather 60 in the illustrated example, a root side portion 60B rises obliquely toward the center in the width direction WD, and a tip side portion 60A, which is closer to a tip portion of the rising gather 60 than an intermediate portion thereof, rises obliquely toward the outside in the width direction. However, a structure of the rising gather 60 is not limited to this example and it can be changed as appropriate, for example, the rising gather 60 rises, as a whole, toward the center in the width direction.

More specifically, the rising gather 60 in the illustrated example is formed as follows. That is, at a portion to be a tip of the rising gather 60, a belt-shaped gather sheet 62, which has a length equal to the length of the inner member 200 in the front-back direction, is folded back in the width direction WD to be folded in two such that a sheet-folded portion is formed at the tip of the rising gather 60, and then, a plurality of elongated gather elastic members 63, which are arranged along a longitudinal direction in a stretched state at intervals in the width direction WD, is fixed between two sheets of the sheet-folded portion and a vicinity thereof. A base portion of the rising gather 60 opposite to the tip portion thereof (an end portion opposite to the sheet-folded portion in the width direction WD) refers to a root portion 65 fixed to the side of the inner member 200, and a portion other than the root portion 65 refers to a main unit portion 66 (a portion on a folded portion side) extending from the root portion 65. In addition, the main unit portion 66 has the root side portion 60B, which extends toward the center in the width direction, and the tip side portion 60A, which is folded back at a tip of the root side portion 60B so as to extend toward the outside in the width direction. Then, a front end portion and a back end portion of the main unit portion 66 refer to fallen portions 67 fixed to a front surface of the side portion of the top sheet 30 in a state of falling down. Meanwhile, an intermediate portion located between the fallen portions 67 in the front-back direction refers to the non-fixed rising portion 68, and the gather elastic members 63 extending along the front-back direction LD are fixed in a stretched state to at least a tip portion of the rising portion 68.

In the rising gathers 60 configured as described above, as indicated by arrows in Fig. 3, the rising portions 68 rise so as to come into contact with a wearer's body surface by contraction forces of the gather elastic members 63. In particular, when the root portions 65 are located on an underside of the inner member 200, since the rising portions 68 rise so as to open toward the outside in the width direction in the crotch part and the vicinity thereof, the rising gathers 60 are brought into surface contact with the peripheries of the wearer's legs and the fitting property of the crotch part is thereby improved. The root portions 65 may be fixed to a top side of the inner member 200, e.g., a top surface of both the side portions of the top sheet 30.

As in the rising gather 60 in the illustrated example, in a bending structure where the main unit portion 66 includes the root side portion 60B, which extends toward the center in the width direction, and the tip side portion 60A, which is folded back at the tip of the root side portion 60B so as to extend toward the outside in the width direction, in each of the fallen portions 67, the tip side portion 60A and the root side portion 60B are bonded to each other in the state of falling down, and the root side portion 60B is bonded to the top sheet 30 in the state of falling down. In the fallen portion 67, for bonding facing surfaces to each other, at least one of a hot melt adhesive by various application methods and a means by material welding such as heat sealing or ultrasonic sealing can be used. In this case, bonding the root side portion 60B and the top sheet 30 to each other and bonding the tip side portion 60A and the root side portion 60B to each other may be performed by the same means or by different means. For example, it is preferable that the root side portion 60B and the top sheet 30 are bonded to each other by the hot melt adhesive and the tip side portion 60A and the root side portion 60B are bonded to each other by material welding.

As the gather sheet 62, a product obtained by subjecting a soft nonwoven fabric having excellent uniformity and concealability, such as a spunbonded nonwoven fabric (SS, SSS, or the like), an SMS nonwoven fabric (SMS, SSMMS, or the like), or a melt blown nonwoven fabric, to a water repellent treatment with silicone or the like as necessary can be used suitably. In this case, the nonwoven fabric preferably has a fiber basis weight of about 10 to 30 g/m². Alternatively, it is possible to use the gather sheet 62 folded in two so as to form two sheets, between which waterproof film is disposed, although not illustrated.

As the gather elastic member 63, a rubber thread or the like can be used. When a spandex rubber thread is used, the spandex rubber thread preferably has a fineness of 470 to 1240 dtex, and more preferably 620 to 940 dtex. The rubber thread preferably has a stretch rate of 150 to 350%, preferably 200 to 300% in a state where the gather elastic member 63 is fixed. The number of the gather elastic members 63 is preferably 2 to 6, and more preferably 3 to 5. A disposition interval of the gather elastic members 63 is suitably 3 to 10 mm. With such a configuration, a range where the gather elastic members 63 are disposed easily comes into surface contact with the wearer's body surface. The gather elastic members 63 may be disposed not only on a tip side but also on a root side.

In the rising portion 68 of the rising gather 60, at least one of a hot melt adhesive by various application methods and a means by material welding such as heat sealing, ultrasonic sealing, etc. can be used for adhering an internal layer of the gather sheet 62 and an external layer of the gather sheet 62 to each other or fixing the gather elastic members 63 interposed therebetween. It is preferred that the portions other than bonded portions of the gather elastic members 63 not be bonded or be weakly bonded, because adhering of the entire faces each other of the inner layer and the outer layer of the gather sheet 62 impairs flexibility. The illustrated example has the following structure. That is, the hot melt adhesive is applied only to outer peripheral surfaces of the gather elastic members 63 using application means such as comb gun or Surewrap nozzle, etc. and the gather elastic members 63 are interposed between the inner layer and the outer layer of the gather sheet 62, and by doing so, with only the hot melt adhesive applied to the outer peripheral surfaces of the gather elastic members 63, fixing of the gather elastic members 63 to the inner layer and the outer layer of the gather sheet 62 as well as fixing the inner layer and the outer layer of the gather sheet 62 can be performed.

Similarly, for fixing the fallen portion 67, at least one of a hot melt adhesive by various application methods and a means by material welding such as heat sealing or ultrasonic sealing can be used.

### (Side flap)

As illustrated in Fig. 1 to Fig. 4 and the like, on both the sides of the inner member 200, side flaps 70 protrude laterally from the absorber 56 and side stretchable regions SG stretching and contracting in the front-back direction are preferably formed in the side flaps 70 respectively. The side flap 70 in the illustrated example includes one or a plurality of mutually spaced elongated side elastic members 73 which are arranged along the front-back direction LD, a first sheet layer 71 which faces an external side of the side elastic member 73, and a second sheet layer 72 which faces an internal side of the side elastic member 73.

Sheet materials forming the first sheet layer 71 and the second sheet layer 72 are not particularly limited, and it is possible, as the sheet materials, to select appropriate nonwoven fabrics such as those available for the above mentioned rising gather 60 and the above mentioned outer member 12F, 12B. In the illustrated example, the gather sheet 62 of the rising gather 60 extends laterally so as to form the first sheet layer 71 and the second sheet layer 72, as discussed later. In this case, the front and back ends of the side flap 70 correspond to the front and back ends of the rising gather 60 (that is, in this case, front and back ends of the inner member 200).

As the side elastic member 73, an elongated elastic member similar to the above mentioned gather elastic member 63 may be used, without any particular limitation. The side elastic member 73 preferably has a stretch rate of 150 to 350%, and more preferably 200 to 270% in a state where the side elastic member 73 is fixed. The number of the side elastic members 73 is preferably 2 to 16, and more preferably 6 to 10. A disposition interval of the side elastic members 73 is suitably 5 to 10 mm.

The side elastic member 73 is fixed to the first sheet layer 71 and the second sheet layer 72. A hot melt adhesive HM by various application methods and a means by material welding such as heat sealing, ultrasonic sealing, etc. can be used for adhering of the first sheet layer 71 and the second sheet layer 72 to each other or fixing of the side elastic member 73 interposed therebetween. It is preferred that the portions other than a bonded portion of the side elastic member 73 not be bonded or be weakly bonded, because a large area of a portion where the first sheet layer 71 and the second sheet layer 72 are bonded to each other impairs flexibility. The illustrated example has the following structure. That is, the hot melt adhesive HM is applied only to an outer peripheral surface of the side elastic member 73 using application means such as comb gun or Surewrap nozzle, etc. and the side elastic member 73 is interposed between the first sheet layer 71 and the second sheet layer 72, and by doing so, with only the hot melt adhesive HM applied to the outer peripheral surface of the side elastic member 73, fixing of the side elastic member 73 to the first sheet layer 71 and the second sheet layer 72 as well as fixing the first sheet layer 71 and the second sheet layer 72 can be performed.

Further, in the illustrated example, in the side flap 70, a sheet material forming the first sheet layer 71 and a sheet material forming the second sheet layer 72 are folded back at a side edge of the side flap 70 to form a folded portion, which is fixed to an underside surface of the liquid impervious sheet 11 (in an enveloping structure). As in the illustrated example, such fixing can be performed by the hot melt adhesive HM. Alternatively, it can be performed also by a material welding.

The side flaps 70 can be omitted.

### (Outer Member)

The outer member 12F, 12B is composed of the front outer member 12F, which is a rectangular shaped member forming at least the lower torso portion in the front body F, and the back outer member 12B, which is a rectangular shaped member forming at least the lower torso portion in the back body B respectively. The front outer member 12F and the back outer member 12B may not continuous on a crotch side but may be separated in the front-back direction LD (outer member separated type), or the front outer member 12F and the back outer member 12B may extend continuously from the front body to the back body (outer member integrated type), although not illustrated. In an outer member separated type underpants type disposable diaper, the separation distance 12d in the front-back direction LD can be set to, for example, about 40 to 60% of the maximum length Y of the diaper. In the illustrated example, lower edge of the front outer member 12F and lower edge of the back outer member 12B may be linear shaped along the width direction WD. However, at least one of the lower edge of the front outer member 12F and the lower edge of the back outer member 12B may be curved so as to follow peripheries of the wearer's legs.

In the outer member separated type underpants type disposable diaper, since the inner member 200 is exposed between the front outer member 12F and the back outer member 12B, in order to prevent the liquid impervious sheet 11 from being exposed on the underside surface of the inner member 200, the cover nonwoven fabric 13 is favorably provided that covers the underside surface of the inner member 200 from a space between the front outer member 12F and the inner member 200 to a space between the back outer member 12B and the inner member 200. An internal surface of the cover nonwoven fabric 13 and an external surface of the cover nonwoven fabric 13 can be bonded to respective facing surfaces by the hot melt adhesive. A nonwoven fabric used for the cover nonwoven fabric 13 can be selected as appropriate, for example, a nonwoven fabric similar to the nonwoven fabric forming the outer member 12F, 12B. Note that the outer member may be continuous from the front body F to the back body B through the crotch part M, although not illustrated. In this case, the outer member may have not only a part corresponding to the lower torso region T but also a part corresponding to the intermediate region L.

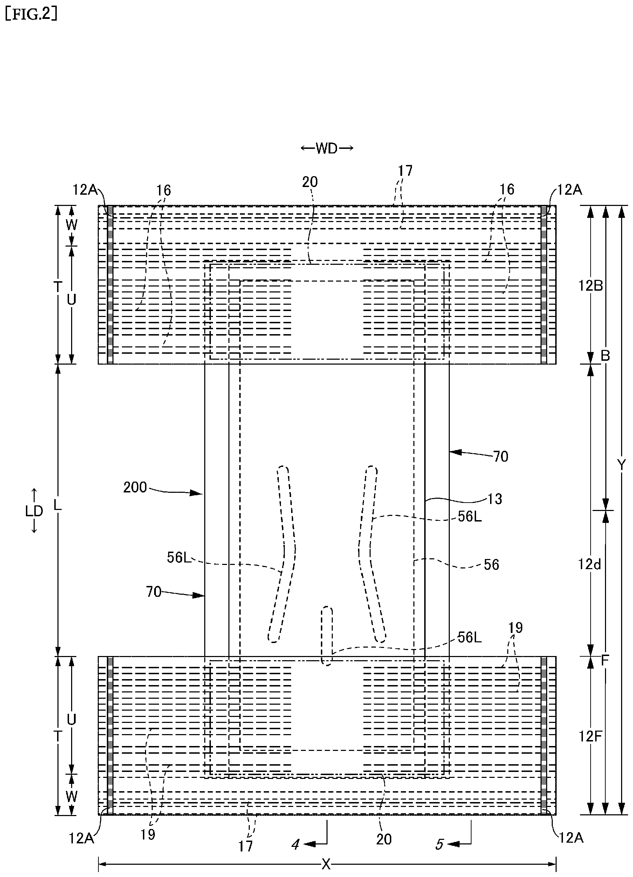

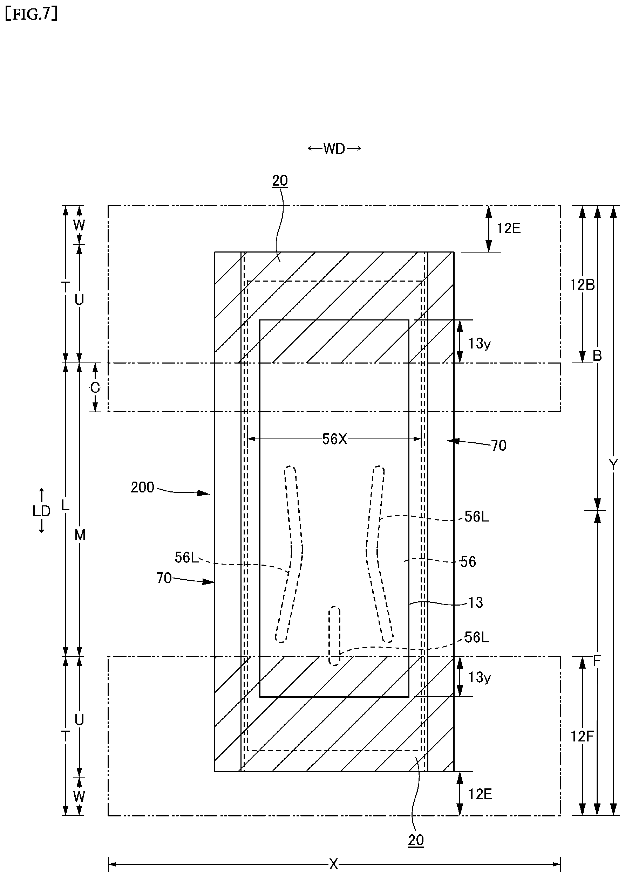

The front outer member 12F and the back outer member 12B have, on the front side and the back side, the lower torso portion forming the lower torso region T. In the example illustrated in Fig. 1 and Fig. 2, a dimension of the front outer member 12F is the same as a dimension of the back outer member 12B in the front-back direction L, and the front outer member 12F and the back outer member 12B do not have a part corresponding to the intermediate region L. Meanwhile, as illustrated in Fig. 7, it is possible that a dimension of the back outer member 12B is longer than a dimension of the front outer member 12F in the front-back direction LD, and the front outer member 12F does not have a part corresponding to the intermediate region L, but the back outer member 12B has a gluteal cover portion C, which protrudes from the lower torso region T so as to extend in the intermediate region L. The front outer member 12F may have also an inguinal cover portion protruding from the lower torso region T so as to extend in the intermediate region L, although not illustrated.

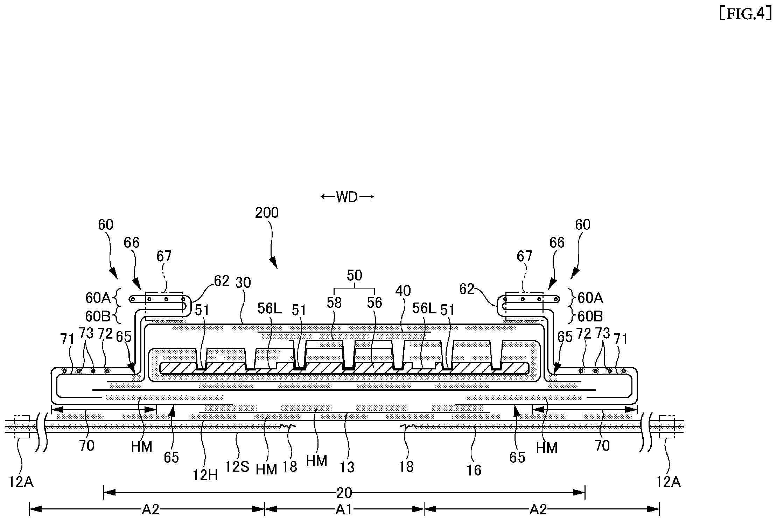

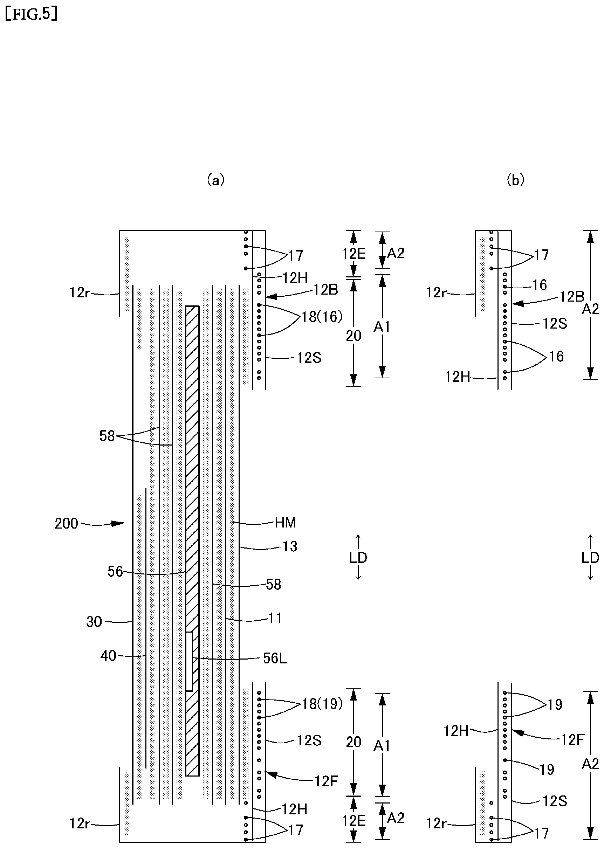

As illustrated in Fig. 4 and Fig. 5, the outer member 12F, 12B is formed by bonding an outer sheet layer and an inner sheet layer, which are adjacent to elastic members 16 to 19 described later on an outer side and an inner side thereof respectively, by a bonding means such as a hot melt adhesive or welding. The outer sheet layer and the inner sheet layer may be composed of two individual sheet materials 12S, 12H, as in the illustrated example. Alternatively, the outer sheet layer and the inner sheet layer may be composed of a common single sheet material. In this latter case, for example, the inner sheet layer and the outer sheet layer are formed by an inner portion and an outer portion of a single sheet of a sheet material folded back at an edge of the waist opening WO (which may be an edge of the lower torso region on the crotch side) in a part or whole of the outer member 12F, 12B respectively. The illustrated example corresponds to an example in that former case, and the sheet material 12S forming the outer sheet layer in the under-waist end portion goes around the sheet material 12H forming the inner sheet layer in the under-waist end portion on the waist opening WO side and folded back inward. An folded-back portion 12r extends so as to cover an end portion of the inner member 200 on the waist opening WO side. Meanwhile, in the waist end portion, the folded-back portion 12r corresponds to an inner sheet layer adjacent to the elastic members on the inner side thereof.

In the outer member 12F, 12B, in order to enhance a fitting property thereof to a wearer's lower torso, the elastic members 16 to 19 are included, and a stretchable region A2, which elastically stretches and contracts in the width direction WD along with stretching and contracting of the elastic members 16 to 19, is formed. In the stretchable region A2, in a natural length state, the outer member 12F, 12B contracts along with contraction of the elastic members to form wrinkles or pleats. When the outer member 12F, 12B stretches in a longitudinal direction of the elastic members, the outer member 12F, 12B can be stretched to a predetermined stretch rate at which the outer member 12F, 12B is spread without wrinkles. As each of the elastic members 16 to 19, in addition to an elongated elastic member (illustrated example) such as a rubber thread, a known elastic member such as a belt-shaped member, a net-shaped member, or a film-shaped member can be used without particular limitation. Each of the elastic members 16 to 19 may be formed of either a synthetic rubber or a natural rubber may be used.

The elastic members 16 to 19 in the illustrated example will be described in more detail. In the waist end portion W of the outer member 12F, 12B, a plurality of waist end portion elastic members 17 is arranged at intervals in the front-back direction so as to be continuous over the entire width direction WD. Among the waist end portion elastic members 17, one or more waist end portion elastic members 17 disposed in a region adjacent to the under-waist end portion U may overlap with the inner member 200, or may be disposed on both sides thereof in the width direction except for the center in the width direction overlapping with the inner member 200. As the waist end portion elastic members 17, it is preferable to dispose about 2 to 15, particularly about 4 to 10 rubber threads each having a fineness of about 155 to 1880 dtex, particularly about 470 to 1240 dtex (in a case of a synthetic rubber, and having a cross section of about 0.05 to 1.5 mm², particularly about 0.1 to 1.0 mm² in a case of a natural rubber) at intervals of 2 to 12 mm, particularly 3 to 7 mm. A resultant stretch rate of the waist end portion W in the width direction WD is preferably about 150 to 400%, and particularly preferably about 220 to 320%. In the waist end portion W, all of the elastic members 17 in the front-back direction LD do not have to have the same fineness and do not have to have the same stretch rate. For example, the fineness of the elastic members 17 partly may be different each other and the stretch rate thereof partly may be different each other.

In addition, in the under-waist end portion U of the outer member 12F, 12B, a plurality of elongated under-waist end portion elastic members 16 and 19 is arranged at intervals in the front-back direction so as to form an under-waist end stretchable region (a region having the under-waist end portion elastic members 16 and 19). As the under-waist end portion elastic members 16 and 19, it is preferable to dispose about 5 to 30 rubber threads each having a fineness of about 155 to 1880 dtex, particularly about 470 to 1240 dtex (in a case of a synthetic rubber, and having a cross section of about 0.05 to 1.5 mm², particularly about 0.1 to 1.0 mm² in a case of a natural rubber) at intervals of 1 to 15 mm, particularly 3 to 8 mm. A resultant stretch rate of the under-waist end portion U in the width direction WD is preferably about 200 to 350%, and particularly preferably about 240 to 300%. In the under-waist end portion U, all of the elastic members in the front-back direction LD do not have to have the same fineness and do not have to have the same stretch rate and the fineness of the elastic members partly may be different each other and the stretch rate thereof partly may be different each other.

In a case where the elastic members 16 and 19 are disposed in a range in the front-back direction having the absorber 56 as in the under-waist end portion U in the illustrated embodiment, in order to prevent contraction of the absorber 56 in the width direction WD in a part or the whole thereof, it is preferable that, as illustrated in Fig. 4 and Fig. 5, intermediate portions in the width direction including a part or whole of a portion overlapping with the absorber 56 in the width direction WD are formed to be a non-stretchable region A1, and both side portions of the front body part and the back body part of the non-stretchable region A1 in the width direction are formed to be the stretchable region A2 (an under-waist end portion stretchable region in the illustrated example). Dimensions in the width direction of the stretchable region A2 disposed at both the sides of non-stretchable region A1 in the width direction can be substantially constant in the front-back direction LD respectively as in the illustrated example. Alternatively, these dimensions can be changed in the front-back direction LD, although not illustrated. In addition, the dimensions in the width direction WD of the stretchable region A2 disposed at both the sides of the front body part of the non-stretchable region A1 in the width direction WD can be substantially the same as or different from the dimensions in the width direction WD of the stretchable region A2 disposed at both the sides of the back body part of the non-stretchable region A1 in the width direction WD.

Such stretchable region A2 and the non-stretchable region A1 can be formed as follows. That is, elastic members 16 to 17 and 19 are fixed between the inner sheet layer and the outer sheet layer, and after that, , the elastic members 16, 19 are cut at one place or over the whole in an intermediate portion in the width direction in a region to be the non-stretchable region A1, by means of cutting or by means of pressing and heating of the elastic members 16, 19 so as to destroy elasticity in the non-stretchable region A1, while in a region to be the stretchable region A2, elastic members 16, 19 are left as they are so as not to destroy elasticity in the stretchable region A2. Meanwhile, it turns out that idle elastic members 18, which do not substantially contribute to generate elasticity, are remained in the stretchable region A2.

As the sheet material 12S forming the inner sheet layer and the sheet material 12H forming the outer sheet layer, any materials can be used without particular limitation. However, a nonwoven fabric is preferably used. In a case where the nonwoven fabric is used, a basis weight per one sheet thereof is preferably about 10 to 30 g/m².

The elastic members 16 to 19 can be fixed to the outer member 12F, 12B by a known method. In addition, the inner sheet layer and the outer sheet layer can be also bonded to each other by a known method. For example, in portions having the elastic members 16 to 19 in the outer member 12F, 12B, the hot melt adhesive HM is applied only to an outer peripheral surface of each of the elastic members 16 to 19 using application means such as comb gun or SureWrap nozzle, etc. and the elastic members 16 to 19 are interposed between the inner sheet layer and the outer sheet layer, and by doing so, with only the hot melt adhesive HM applied to the outer peripheral surfaces of the elastic members 16 to 19, fixing of the elastic members 16 to 19 to the inner sheet layer and the outer sheet layer as well as fixing the first sheet layer and the second sheet layer can be performed.

### (Inner and outer bonded portion)

The inner member 200 can be fixed to the outer member 12F, 12B by a bonding means by material welding such as heat sealing or ultrasonic sealing, or by the hot melt adhesive. In the illustrated example, via the hot melt adhesive applied to the underside surface of the inner member 200, that is, in this case, to the underside surface of the liquid impervious sheet 11 and root portions 65 of the rising gather 60, the inner member 200 is fixed to the inner surface of the outer member 12F, 12B. An inner and outer bonded portion 20 for fixing the inner member 200 to the outer member 12F, 12B can be disposed in almost the entire region where the inner member 200 overlaps with the outer member 12F, 12B as illustrated in Fig. 2. Alternatively, the inner and outer bonded portion 20 can be disposed, for example, in a portion excluding both end portions of the inner member 200 in the width direction.

### <Description of Terms Used in Specification>

The following terms used in the specification have the following meanings unless otherwise specified in the specification. [0090]
- "Front-back direction" refers to a direction (longitudinal direction) indicated by a reference sign LD in the drawings, "width direction" means a direction (left-right direction) indicated by a reference sign WD in the drawings, and the front-back direction and the width direction are orthogonal to each other.
- "Top side" refers to a side closer to a wearer's skin when a disposable diaper is worn, and "underside" means a side far from a wearer's skin when a disposable diaper is worn.
- "Top surface" refers to a surface of a portion closer to a wearer's skin when a disposable diaper is worn, and "underside surface" refers to a surface of a portion far from the wearer's skin when the disposable diaper is worn.
- "Stretch rate" refers to a value when the natural length is 100%. For example, a stretch rate of 200% is synonymous with an elongation ratio of 2.
- "Gel strength" is measured as follows. 1.0 g of super absorbent polymers is added to 49.0 g of artificial urine (urea: 2 wt%, sodium chloride: 0.8 wt%, calcium chloride dihydrate: 0.03 wt%, magnesium sulfate heptahydrate: 0.08 wt%, and ion exchanged water: 97.09 wt%) and the mixture is agitated with a stirrer. The resulting gel is left in a thermo-hygrostat at 40°C × 60%RH for three hours and then cooled to room temperature. The gel strength of the gel is measured with a curdmeter (Curdmeter-MAX ME-500 manufactured by I. Techno Engineering Co., Ltd).
- "Basis weight" is measured as follows. A sample or a test piece is pre-dried, and then is left in a test room or a device in a standard state (temperature 23 ± 1°C, relative humidity 50 ± 2% in a test location), and is put in a constant weight state. Pre-drying refers to setting the weight of the sample or the test piece to a constant weight in an environment in which temperature is 100°C. Incidentally, pre-drying is unnecessary for a fiber having an official moisture regain of 0.0%. A sample having dimensions of 100 mm × 100 mm is cut off from the test piece in the constant weight state using a sampling template (100 mm × 100 mm). A weight of the sample is measured and multiplied by 100 to calculate a weight per square meter, and the weight is set to the basis weight.
- The "thickness" of a thick member such as the absorber 56, the absorbent element 50, or the compressed portion 51 is measured using a thickness measuring instrument of Ozaki Mfg. Co., Ltd. (Peacock, Dial Thickness Gauge, Model H (measurement range of 0 to 10 mm, circular contact point with a diameter of 10 mm, measuring force of about 1.7 N, and pressure of about 21.7 KPa) by horizontally placing the sample and the thickness measuring device.
- The "thickness" of a thin member such as a nonwoven fabric is automatically measured under the condition of load: 0.098 N/cm² and pressure area: 2 cm² using an automatic thickness meter (KES-G5 handy compression measurement program).
- Water absorption capacity is measured in accordance with JIS K7223-1996 "Testing method for water absorption capacity of super absorbent polymers."
- Water absorption rate is defined as "time that elapse before the end point" measured with 2g of super absorbent polymers and 50g of normal saline solution in accordance with JIS K7224-1996 "Testing method for water absorption rate of super absorbent polymers."
- "Spread state" means a flatly spread state without contraction (including any contraction such as one due to an elastic member) or slack.
- The dimensions of the respective components are measured in a spread state, not the natural length state, unless otherwise specified.
- The tests and measurements are carried out in a test room or a test device under normal conditions (an ambient temperature of 23 ± 1°C and with a relative humidity of 50 ± 2% at the testing site), unless the environmental condition for the tests and measurements are otherwise specified.

### Industrial Applicability

In particular, the present invention can be applied as appropriate for an underpants type disposable diaper from the view point of importance of solution for solving the above problems, in addition, the present invention can be applied also for a general underpants-type disposable diaper such as a tape-type disposable diaper or a pad type disposable diaper.

### Reference Signs List

- 11: Liquid impervious sheet
- 12A: Side seal portion
- 12B: Back outer member
- 12E: Waist opening side extending portion
- 12F, 12B: Outer member
- 12F: Front outer member
- 12S, 12H: Sheet material
- 13: Cover nonwoven fabric
- 16, 19: Under-waist end portion elastic member
- 17: Waist end portion elastic member
- 18: Idle elastic member
- 20: Inner and outer bonded portion
- 200: Inner member
- 30: Top sheet
- 40: Intermediate sheet
- 50: Absorbent element
- 51: Compressed portion
- 51f: Unit frame
- 56: Absorber
- 56L: Low-basis weight section
- 58: Wrapping sheet
- 60: Rising gather
- 60A: Tip side portion
- 60B: Root side portion
- 62: Gather sheet
- 63: Gather elastic member
- 67: Fallen portion
- 68: Rising portion
- 70: Side flap
- 71: First sheet layer
- 72: Second sheet layer
- 73: Side elastic member
- A1: Non-stretchable region
- A2: Stretchable region
- B: Back body
- C: Inguinal cover portion
- F: Front body
- HM: Hot melt adhesive
- L: Intermediate region
- LD: Front-back direction
- LO: Leg opening
- M: Crotch part
- SG: Side stretchable region
- SR: region being lateral to a low-basis weight section
- T: Lower torso region
- U: Under-waist end portion
- W: Waist end portion
- WD: Width direction
- WO: Waist opening
- HM1: First hot melt adhesive
- HM2: Second hot melt adhesive

## Claims

1. A disposable diaper comprising:
a crotch part;
a front side part which extends forward from the crotch part;
a back side part which extends backward from the crotch part; and
an absorbent element including an absorber and a wrapping sheet wrapping the absorber, the absorbent element being provided in a range in a front-back direction including the crotch part,
wherein the absorber is formed by accumulating a mixture of pulp fibers and super absorbent polymer particles,
in the crotch part, the absorber has elongated low-basis weight sections, which extend in the front-back direction, on both sides thereof in a width direction,
compressed portions, which are compressed in a thickness direction so as to be depressed from at least one of a top surface and an underside surface of the absorbent element into the absorber, are continuous in lattice shaped patterns provided across at least entire regions being lateral to the low-basis weight sections in the absorbent element, and
the wrapping sheet is made of a nonwoven fabric having a bending resistance of 0.01 to 0.10 mN·cm in the front-back direction and 0.01 to 0.10 mN·cm in the width direction according to 41.5° Cantilever Method defined by JIS L 1913: 2010.

2. The disposable diaper according to claim 1,
wherein the absorber has, in the crotch part, a narrowest portion having a width being 0.85 times to 1 time a maximum width of the front side part and the back side part.

3. The disposable diaper according to claim 1 or 2,
wherein, in the absorber, the pulp fibers have a basis weight of 100 to 450 g/m², and
in the absorber, weight ratio of the pulp fibers : the super absorbent polymer particles is 50 : 50 to 20 : 80.

4. The disposable diaper according to claim 3,
wherein a width of each of the compressed portions is 1 to 3 mm, a thickness of each of the compressed portions is 15 to 35% of a maximum thickness of the absorbent element,
the compressed portions are formed in an oblique lattice including first portions each of which diagonally inclines at a clockwise angle of 40 to 50° with respect to the front-back direction in a plan view, and second portions each of which diagonally inclines at a counterclockwise angle of 40 to 50° with respect to the front-back direction in the plan view,
two or more unit frame rows in each of which unit frames including the compressed portions are repeated in the front-back direction are arranged in the width direction in at least a part of each of the regions being lateral to the low-basis weight sections.

5. The disposable diaper according to claim 4,
wherein across at least entire the regions being lateral to the low-basis weight sections, an internal surface of the wrapping sheet is bonded to at least one of a top surface and an underside surface of the absorber on a side thereof, which has grooves of the compressed portions, by 5.0 to 20.0 g/m² of a hot melt adhesive.

6. The disposable diaper according to any one of claims 1 to 5,
wherein the wrapping sheet has
an elongation rate of 20 to 100% in the front-back direction, measured according to a normal time method specified in JIS L 1913 : 2010, and
an elongation rate of 50 to 110% in the width direction, measured according to the normal time method specified in JIS L 1913 : 2010.
